# EUROPEAN PATENT APPLICATION

(11) **EP 4 249 508 A1**
(43) Date of publication of application: **27.09.2023**
(21) Application number: 21894611.9
(22) Date of filing: 16.11.2021
(51) Int. Cl.: C07K 16/10, C12N 7/00, G01N 33/543, G01N 33/569

(54) **SARS-COV-2 DETECTION KIT AND SARS-COV-2 DETECTION METHOD**

(30) Priority: 17.11.2020 JP 2020190706
(71) Applicant: FUJIFILM Corporation, Tokyo 106-8620 (JP); Public University Corporation Yokohama City University, Yokohama-shi, Kanagawa 236-0027 (JP)
(72) Inventor: UJIHARA Dai, Ashigarakami-gun, Kanagawa 258-8538 (JP); KATADA Junichi, Ashigarakami-gun, Kanagawa 258-8538 (JP); RYO AKIHIDE, Yokohama-shi, Kanagawa 236-0004 (JP)
(74) Representative: Owen, Deborah Jane
(86) International application number: PCT/JP2021/041983
(87) International publication number: WO 2022/107737

(57) **Abstract**

An object of the present invention is to provide a SARS-CoV-2 detection kit and a SARS-CoV-2 detection method which make it possible to simply detect SARS-CoV-2 with higher sensitivity. According to the present invention, there is provided a SARS-CoV-2 detection kit which is for specifically detecting a nucleocapsid protein contained in a biological specimen and contains at least one antibody that specifically reacts with a SARS-CoV-2 nucleocapsid protein (NP), the SARS-CoV-2 detection kit including a first container that houses a silver-containing compound, and a second container that houses a reducing agent capable of reducing silver ions, in which the antibody includes at least one antibody that belongs to a subclass IgG2b.

## Description

### Field of the Invention

The present invention relates to a SARS-CoV-2 detection kit and a SARS-CoV-2 detection method which utilize silver amplification of detection information.

### Description of the Related Art

As a main technique of diagnosing severe acute respiratory syndrome (SARS)-CoV-2, which is the causative virus of COVID-19, that caused a pandemic in 2020, for example, a reverse transcription polymerase chain reaction (RT-PCR) for detecting genomic RNA is widely marketed as described in Non-Patent Document 1. In the detection by RT-PCR, a trace of RNA is converted into cDNA by using a reverse transcriptase and then amplified to a detectable concentration, which makes it possible to detect a small amount of RNA. RT-PCR is a test method capable of making a definite diagnosis with high sensitivity.

Incidentally, as described in Non-Patent Document 2, an antigen detection kit has been developed which is for detecting nucleocapsid protein (NP) known to have a function of protecting genomic RNA and for diagnosing COVID-19. The antigen test is a test method of mainly detecting the presence of the contained protein together with the SARS-coronavirus gene. Among such test methods, a detection method using immunochromatography which enables a simple test is used.

Patent Document 1 describes a nonhuman animal-derived antiserum and/or a nonhuman animal-derived immunoglobulin against the 2019-nCOV (SARS-CoV-2) virus.

Patent Document 2 describes a monoclonal antibody against a novel coronavirus or a derivative thereof, the monoclonal antibody having identified antigen complementarity-determining regions CDR1, CDR2, and CDR3.

Among the testing methods using an immune reaction, immunochromatography is generally widely used by medical practitioners and physicians in clinics because this method enables a simple and rapid test. Patent Document 3 discloses, as a method of enhancing sensitivity of the immunochromatography, a technique of enabling detection with high sensitivity by increasing the size of labeled particles by using a silver-containing compound and a reducing agent for silver ions.

### Prior Art Documents

### Patent Documents

Patent Document 1: CN111471103A
Patent Document 2: CN111560070A
Patent Document 3: JP5091075B
Non-Patent Documents

Non-Patent Document 1: National Institute of Infectious Diseases, Japan, Pathogen detection manual 2019-nCOV Ver.2.9.1
Non-Patent Document 2: Japanese Journal of Medicine and Pharmaceutical Science, Vol. 77, No. 6, Development of Novel Coronavirus SARS-CoV-2 antigen detection reagent using immunochromatography, SARS-CoV-2

### SUMMARY OF THE INVENTION

As described above, the test by RT-PCR, which is a method of detecting genomic RNA information, has higher sensitivity compared to the antigen test and is capable of making a definite diagnosis. However, RT-PCR requires a long testing time, which makes it difficult to rapidly obtain a result. On the other hand, the detection method using immunochromatography as an antigen test is a simple test method capable of performing a test in a short time. However, it has been pointed out that such a detection method has lower sensitivity compared to the RT-PCR test.

An object of the present invention is to provide a SARS-CoV-2 detection kit and a SARS-CoV-2 detection method which make it possible to simply detect SARS-CoV-2 with higher sensitivity.

As a result of conducting intensive studies to achieve the above object, the inventors of the present invention have found that using an antibody for detecting a SARS-CoV-2 nucleocapsid protein (NP), a silver-containing compound, and a reducing agent capable of reducing silver ions and amplifying information of the SARS-CoV-2 nucleocapsid protein (NP) detected by the antibody makes it possible to simply perform a test with high sensitivity. Based on this finding, the inventors have accomplished the present invention.

That is, according to an aspect of the present invention, the following inventions are provided.
<1> A SARS-CoV-2 detection kit for specifically detecting a nucleocapsid protein (NP) contained in a biological specimen, containing at least one antibody that specifically reacts with a SARS-CoV-2 nucleocapsid protein (NP), the SARS-CoV-2 detection kit including;
   a first container that houses a silver-containing compound; and
   a second container that houses a reducing agent capable of reducing silver ions,
   in which the antibody includes at least one antibody that belongs to a subclass IgG2b.
<2> The SARS-CoV-2 detection kit described in <1>, in which the antibody is a fragmented antibody.
<3> The SARS-CoV-2 detection kit described in <2>, in which the fragmented antibody is an antibody prepared by a protease treatment.
<4> The SARS-CoV-2 detection kit described in any one of <1> to <3>, in which the antibody is labeled with a labeling substance, and information from the labeling substance is detected by being amplified with the silver-containing compound and the reducing agent capable of reducing silver ions.
<5> The SARS-CoV-2 detection kit described in <4>, in which the labeling substance is colloidal gold.
<6> The SARS-CoV-2 detection kit described in any one of <1> to <5>, in which the reducing agent is a metal salt of Fe²⁺.
<7> The SARS-CoV-2 detection kit described in any one of <1> to <6>, further including an insoluble carrier.
<8> The SARS-CoV-2 detection kit described in <7>, in which the insoluble carrier has a capture region for capturing a test substance and a color developing region for detecting the reducing agent.
<9> A SARS-CoV-2 detection method for specifically detecting a nucleocapsid protein (NP) contained in a biological specimen by using at least one antibody that specifically reacts with a SARS-CoV-2 nucleocapsid protein (NP), the SARS-CoV-2detection method including:
   amplifying detection information of the nucleocapsid protein (NP) by using a silver-containing compound and a reducing agent capable of reducing silver ions,
   in which at least one antibody that belongs to a subclass IgG2b is used as the antibody.
<10> The SARS-CoV-2 detection method described in <9>, in which the antibody is a fragmented antibody.
<11> The SARS-CoV-2 detection method described in <10>, in which the fragmented antibody is an antibody prepared by a protease treatment.
<12> The SARS-CoV-2 detection method described in any one of <9> to <11>, in which the antibody is labeled with a labeling substance, and information from the labeling substance is detected by being amplified with the silver-containing compound and the reducing agent capable of reducing silver ions.
<13> The SARS-CoV-2 detection method described in <12>, in which the labeling substance is colloidal gold.
<14> The SARS-CoV-2 detection method described in any one of <9> to <13>, in which the reducing agent is a metal salt of Fe²⁺.
<15> The SARS-CoV-2 detection method described in any one of <9> to <14>, in which the detection method is immunochromatography.

With the SARS-CoV-2 detection kit and SARS-CoV-2 detection method according to an embodiment of the present invention, it is possible to simply detect SARS-CoV-2 with high sensitivity.

### BRIEF DESCRIPTION OF THE DRAWINGS

Fig. 1 is a perspective view showing an aspect of an embodiment of an immunochromatography kit of the present invention.
Fig. 2 is a schematic exploded perspective view showing an aspect of an embodiment of the immunochromatography kit of the present invention.
Fig. 3 is a schematic lateral view showing the positional relationship between a test strip and first and second pots.

### DESCRIPTION OF THE PREFERRED EMBODIMENTS

Hereinafter, embodiments of the present invention will be specifically described.

In the present invention, a range of numerical values described using "to" means a range including the numerical values listed before and after "to" as the minimum value and the maximum value respectively.

### (1) Corona virus

The novel coronavirus which is a detection target in the present invention is a positive-sense single-stranded RNA virus that belongs to the order Nidovirales and has a large genome having an envelope and a length of about 30 kbp. This virus is named SARS-CoV-2, and the official name that World Health Organization (WHO) has given to the infectious disease caused by this novel coronavirus is "COVID-19". It is known that the proteins constituting SARS-CoV-2 include at least four structural proteins, a spike (S) protein, an envelope (E) protein, a membrane (M) protein, and a nucleocapsid (N) protein. Among these, the nucleocapsid (N) protein is an immunogenic phosphoprotein that functions to package the viral RNA genome in the envelope of the virus into a ribonucleoprotein complex called a capsid and has sequence conservation properties and potent immunogenicity.

### (2) Antigen

The aforementioned four structural proteins can be used as an antigen. In the present invention, owing to its sequence conservation properties and potent immunogenicity as an immunogenic phosphoprotein, the nucleocapsid (N) protein is used as an antigen as a detection target.

### (3) Antibody

The antibody used in the present invention is an antibody against the nucleocapsid (N) protein. Not only this protein, but also general antibodies have subclasses that can be sorted into five classes, IgA, IgD, IgE, IgM, and IgG. Among these, IgG is the most abundant antibody class in serum, and is further classified into five subclasses (IgG1, IgG2a, IgG2b, IgG3, and IgG4). Among these subclasses, as an antibody capable of detecting a nucleocapsid protein (hereinafter, also called NP) serving as an antigen with high sensitivity, at least one kind of anti-NP antibody that belongs to the subclass IgG2b is used in the present invention.

### (4) Antibody production

As the antibody, an antiserum prepared from the serum of an animal immunized with an antigen (test substance) or an immunoglobulin fraction purified from the antiserum may be used. That is, it is possible to use a polyclonal antibody produced by the immunization of a laboratory animal with an antigen.

Furthermore, a monoclonal antibody may be used which is produced from a fused cell (hybridoma) obtained by fusing B cells or a cell group containing B cells collected from the spleen of an animal immunized with an antigen in the abdominal cavity with myeloma cells. Alternatively, a monoclonal antibody may be used which is produced from cells by the introduction of a plasmid into host cells. In the present invention, it is preferable to use a method of producing a monoclonal antibody.

### (5) Antibody fragmentation

It is also preferable to use a fragmented antibody [for example, F(ab')₂, Fab, Fab', or Fv] obtained by the fragmentation of the antibody obtained as described above.

As the fragmented antibody, an antibody prepared by a protease treatment can be used. Typically, the fragmented antibody is prepared by the following two methods. First, in a case where the antibody is treated with a papain enzyme, the antibody is decomposed into two Fab fragments and one Fc fragment. In a case where the antibody is treated with a pepsin enzyme, the antibody is decomposed into F(ab')₂ having two Fab fragments linked together and fragmented Fc fragments. F(ab')₂ can also be converted into Fab' by being treated with a reducing agent such as 2-mercaptoethylamine. In addition to these, there are enzymes producing fragmented antibodies, such as ficin, lysyl endopeptidase, V8 protease, bromelain, clostripain, metalloendopeptidase, activated papain obtained by activating papain, and the like. The Fab fragment, the F(ab')₂ fragment, and the Fab' fragment obtained by the treatment with these enzymes have an antigen binding site, and an unnecessary Fc fragment has been removed from these fragments. Therefore, using these fragments in antigen detection reduces nonspecific adsorption and noise. The fragmented antibody used in the present invention is not limited to the fragmented antibody prepared by the above method, and any fragmented antibody can be used as long as the Fc fragment has been removed from the antibody.

### (6) Immunochromatography

In one example of the present invention, the detection information (detection signal) can be amplified by immunochromatography. Generally, the immunochromatography is a method of simply, rapidly, and specifically identifying and testing a test substance by the following technique. In this method, the information of a labeling substance is used as detection information. More specifically, the method is as below. That is, as a stationary phase, a chromatographic carrier (a part of an insoluble carrier) is used which comprises a labeling substance capture region that includes at least one test region having a binding substance capable of binding to a test substance (corresponding to a second binding substance capable of binding to the test substance or a substance exhibiting binding properties to the first binding substance described below, specifically, an antigen, an antibody, or the like). On the chromatographic carrier, as a mobile phase, a liquid containing a labeling substance modified with the first binding substance capable of binding to the test substance is moved in a chromatographic manner. As a result, the test substance and the labeling substance reach the labeling substance capture region having the test region while specifically binding to each other. In the test region of the labeling substance capture region, a complex of the test substance and the labeling substance specifically binds to an immobilized binding substance (the second binding substance or the substance exhibiting binding properties to the first binding substance), which allows the labeling substance to be concentrated in the binding substance (the second binding substance or the substance exhibiting binding properties to the first binding substance) only in a case where the test substance is present in a test sample. The amount of labels is measured by visual observation or using an appropriate instrument to qualitatively and quantitatively analyze the presence of the test substance in the test sample.

During the immunochromatography in the present invention, after the complex of the test substance and the labeling substance specifically binds to the immobilized binding substance (the second binding substance or the substance exhibiting binding properties to the first binding substance) in the test region of the labeling substance capture region, the reaction site is washed with a washing solution, and then signal amplification is performed using an amplification solution. By using amplification reagents (preferably two amplification reagents, for example, preferably a reducing agent capable of reducing silver ions and a silver-containing compound) that are used for amplifying signals of the labeling substance and using, as a nucleus, the complex of the test substance and the labeling substance having bound to an immobilization reagent on the labeling substance capture region, signal amplification is performed by an amplification reaction, which makes it possible to increase sensitivity. According to the present invention, it is possible to carry out a test with rapid high-sensitivity chromatography.

### (7) Test sample

As a test sample in the detection kit and the detection method according to the embodiment of the present invention, it is possible to use a biological specimen (for example, nasal discharge, nasal mucus, saliva, or the like) containing the SARS-CoV-2 virus which is a novel coronavirus. As the test substance, a substance enabling the detection of the novel coronavirus SARS-CoV-2 is preferable. The nucleocapsid protein (NP), which is a nucleoprotein, can be detected as the test substance. The coronavirus is known to contain at least four structural proteins, a spike (S) protein, an envelope (E) protein, a membrane (M) protein, and a nucleocapsid (N) protein. Among these, the nucleocapsid (N) protein is an immunogenic phosphoprotein that is involved in viral genome replication and control of cell signal transduction. Owing to its sequence conservation properties and potent immunogenicity, the nucleocapsid (N) protein is selected as a test substance for diagnosis.

### (8) Pretreatment of test sample

In the present invention, the test sample can be used as it is or can be used in the form of an extract obtained by extracting the test sample with an appropriate extractant, in the form of a diluted solution obtained by diluting the extract with an appropriate diluent, or in the form of a concentrate obtained by concentrating the extract by an appropriate method. As the extractant used in the present invention, it is possible to use a solvent (for example, water, a saline, a buffer solution, or the like) used in an ordinary immunological assay or a hydrophilic organic solvent capable of directly causing an antigen-antibody reaction by being diluted with the aforementioned solvent.

### (9) Configuration

In a case where the present invention is embodied by immunochromatography, a chromatography strip can be used by being incorporated into an immunochromatography kit. The chromatography strip that can be used is not particularly limited as long as it is a chromatography strip that can be used in normal immunochromatography.

The chromatography strip that can be used in the present invention has a labeling substance-holding region and a labeling substance capture region that are arranged in this order from the upstream side to the downstream side in the development direction of a test sample containing a test substance. For example, an aspect is preferably used in which a sample addition pad, a labeling substance-holding pad having a labeling substance-holding region (for example, a colloidal gold antibody-holding pad), a binding substance-immobilized membrane that is an insoluble carrier (for example, a chromatographic carrier having a labeling substance capture region), and an absorbent pad are arranged in this order on a pressure-sensitive adhesive sheet. The chromatographic carrier (insoluble carrier) on which the binding substance is immobilized includes a labeling substance capture region having at least one test region (also called a test line) that is a reaction site where an antibody or an antigen specifically binding to the test substance is immobilized. As desired, the labeling substance capture region may additionally have a region having at least one confirmation region (also called a control region) where a control antibody or a control antigen is immobilized. Furthermore, an amplification index region may be on the downstream of the labeling substance capture region.

The labeling substance-holding pad having a labeling substance-holding region that can be used in the present invention can be prepared by preparing a suspension containing a labeling substance, applying the suspension to an appropriate absorbent pad (for example, a glass fiber pad), and then drying the absorbent pad.

### (9-1) Labeling substance

As the labeling substance (a label used for labeling the first binding substance that specifically binds to the test substance (antigen)) to be used in the present invention, it is preferable to use a labeling substance containing a metal. The type of metal that can be used in the present invention is preferably a noble metal, such as gold, silver, or platinum, iron, lead, copper, cadmium, bismuth, antimony, tin, or mercury. The metal is more preferably a noble metal such as gold, silver, or platinum. The labeling substance containing a metal that can be used in the present invention is preferably in the form of a colloidal metal label or a metal sulfide label. As the colloidal metal label, colloidal platinum, colloidal gold, colloidal silver, colloidal iron, colloidal aluminum hydroxide, or the like can be preferably used. As the metal sulfide label, sulfides of iron, silver, lead, copper, cadmium, bismuth, antimony, tin, or mercury can be preferably used. In the present invention, colloidal platinum, colloidal gold, or colloidal silver can be more preferably used, and colloidal gold can be most preferably used. In a case where colloidal gold particles are used as a colloidal metal label, commercially available colloidal gold particles may be used. Alternatively, the colloidal gold particles can be prepared by a conventional method, for example, a method of reducing chloroauric acid with sodium citrate (Nature Physical Science, 241 (1973), 20, or the like).

The average particle diameter of the colloidal metal is preferably about 1 nm to 500 nm, more preferably 3 to 100 nm, and particularly preferably 5 to 60 nm. The average particle diameter of the colloidal metal used in the present invention can be measured with a commercially available particle size distribution analyzer or the like. As particle size distribution measurement methods, optical microscopy, confocal laser microscopy, electron microscopy, atomic force microscopy, a static light scattering method, laser diffractometry, a dynamic light scattering method, centrifugal sedimentation, electric pulse sensing, chromatography, an ultrasonic attenuation method, and the like are known, and devices compatible with the principles of these methods are commercially available.

In view of the range of particle diameter and ease of measurement, a dynamic light scattering method can be preferably used in the present invention. Examples of commercially available measurement devices using dynamic light scattering include NANOTRAC UPA (Nikkiso Co., Ltd.), dynamic light scattering-type particle size distribution analyzer LB-550 (HORIBA, Ltd.), a particle size analyzer FPAR-1000 for a concentrated solution (Otsuka Electronics Co., Ltd.), and the like. In the present invention, a median diameter (d = 50) measured at a measurement temperature of 25°C is adopted.

The chromatography using a colloidal metal label, a metal sulfide label, another metal alloy label (hereinafter, called a metal-based label in some cases), or a polymer particle label containing a metal as a labeling substance for detection features the amplification of signals of the metal-based label. Specifically, after the complex of the test substance and the labeling substance is formed, in a case where silver ions supplied from a silver-containing compound, such as an inorganic silver salt or an organic silver salt, are brought into contact with a reducing agent capable of reducing the silver ions such that silver particles are generated by means of the reduction of the silver ions by the reducing agent, the metal-based label serves as a nucleus, and the silver particles are deposited on the metal-based label. Therefore, the metal-based label is amplified, which makes it possible to analyze the test substance with high sensitivity. Accordingly, in the immunochromatography of the present invention, the chromatography known in the related art can be used as it is, except that in the immunochromatography of the present invention, a reaction of causing silver particles to be deposited on the label of an immune complex is performed using silver particles generated by the silver ion-reducing action of the reducing agent, and that the signal amplified in this way is analyzed.

### (9-2) Binding substance

The labeling substance is modified with the first binding substance capable of binding to a test substance. As the first binding substance, for example, it is possible to use any compound having affinity with the test substance, such as an antibody against the test substance (antigen), an antigen against the test substance (antibody), or an aptamer against the test substance (such as a protein or a low-molecular-weight compound). In the present invention, it is preferable to use an antibody that specifically reacts with the SARS-CoV-2 nucleocapsid protein (NP). In the present invention, an antibody that belongs to the antibody subclass IgG2b is used as at least one of the second binding substance or the first binding substance described below.

The detection kit according to the embodiment of the present invention preferably has an insoluble carrier (chromatographic carrier), and a strip of the carrier has a labeling substance capture region. The labeling substance capture region has a test region that has the second binding substance capable of binding to the test substance. In a preferred aspect, the labeling substance capture region additionally has a confirmation region having a binding substance that exhibits binding properties to the first binding substance.

As the second binding substance capable of binding to the test substance, for example, it is possible to use any compound having affinity with the test substance, such as an antibody against the test substance (antigen), an antigen against the test substance (antibody), or an aptamer against the test substance (such as a protein or a low-molecular-weight compound). In the present invention, it is preferable to use an antibody that specifically reacts with the SARS-CoV-2 nucleocapsid protein (NP).

In the present invention, an antibody that belongs to the antibody subclass IgG2b is used as at least one of the first binding substance or the second binding substance described above. In addition, the second binding substance and the first binding substance may be different from each other or may be the same as each other. The substance that exhibits binding properties to the first binding substance may be the test substance or a compound having a site recognized by the first binding substance. Examples of such a substance include a compound that may be a conjugate of a derivative of the test substance and a protein (for example, bovine serum albumin (BSA) or the like) and the like.

It is preferable that either or both of the first binding substance and second binding substance be an antibody. It is more preferable that the first binding substance be an antibody specifically reacting with the SARS-CoV-2 nucleocapsid protein (NP), and the second binding substance be an antibody specifically reacting with the SARS-CoV-2 nucleocapsid protein (NP).

An aspect is also preferable in which the labeling substance capture region of the insoluble carrier of the present invention has an amplification index region. The amplification index region is a region that develops color or changes color by reacting with an amplification reagent that will be described later, such that whether the developed amplification reagent has reached the amplification index region is determined. The amplification index region is preferably a color developing region where a reducing agent is detected. For example, in a case where a mixed aqueous solution of an aqueous iron nitrate solution and citric acid (manufactured by FUJIFILM Wako Pure Chemical Corporation, 038-06925) is used as a second amplification solution, an aspect is preferable in which the amplification index region is configured with a color developing reagent-immobilized line where bromocresol green (manufactured by FUJIFILM Wako Pure Chemical Corporation) is linearly immobilized. In a case where the second amplification solution reaches the amplification index region, the region turns orange from green. Such a color change can be used as an index showing that the amplification index region and the region developed to the amplification index region are fully filled with the second amplification solution.

In the present invention, among the methods of modifying a labeling substance with the first binding substance, for example, as a method of binding a colloidal metal to a specific binding substance, the following method known in the related art (for example, The Journal of Histochemistry and Cytochemistry, 30, 7, (1982) 691-696) can be exemplified. Specifically, for example, colloidal metal and a specific binding substance (for example, an antibody) are mixed together in an appropriate buffer solution at room temperature for 5 minutes or longer. After the reaction, by dispersing the sediment obtained by centrifugation in a solution containing a dispersant such as polyethylene glycol, it is possible to obtain a target substance.

### (9-3) Insoluble carrier

As the insoluble carrier that can be used in the present invention, a porous carrier is preferable. Particularly, a nitrocellulose membrane, a cellulose membrane, an acetylcellulose membrane, a polysulfone membrane, a polyethersulfone membrane, a nylon membrane, glass fiber, nonwoven fabric, cloth, a thread, or the like is preferable, and a nitrocellulose membrane is more preferable.

In the present invention, it is preferable that the labeling substance capture region of the insoluble carrier have the second binding substance capable of binding to the test substance and have a confirmation region where a substance that exhibits binding properties to the first binding substance is immobilized. The second binding substance capable of binding to the test substance or the substance that exhibits binding properties to the first binding substance may be directly immobilized in a part of the insoluble carrier by a physical or chemical bond to form the labeling substance capture region. Alternatively, the second binding substance capable of binding to the test substance or the substance that exhibits binding properties to the first binding substance may be physically or chemically bonded to fine particles such as latex particles, and the fine particles may be trapped and immobilized in a portion of the insoluble carrier to form the labeling substance capture region. The insoluble carrier in which the second binding substance or the substance that exhibits binding properties to the first binding substance has been immobilized is preferably used after being subjected to a non-specific adsorption prevention treatment by a treatment with an inactive protein or the like. It is also possible to preferably use an aspect in which the insoluble carrier of the present invention has a plurality of test regions. Furthermore, as desired, the insoluble carrier of the present invention may have the aforementioned confirmation region as a portion of the labeling substance capture region.

### (9-4) Labeling substance-holding pad

It is preferable that a labeling substance-holding pad having a labeling substance-holding region be used by being incorporated into the insoluble carrier. Examples of the labeling substance-holding pad include a pad that holds colloidal gold. As a material of the labeling substance-holding pad, for example, cellulose filter paper, glass fiber, nonwoven fabric, or the like can be preferably used. The labeling substance-holding pad can be prepared by impregnating the aforementioned material with a certain amount of labeling substance prepared as described above and then drying the material.

### (9-5) Sample addition pad

It is preferable that a sample addition pad be used by being incorporated into the insoluble carrier. For the sample addition pad, an aspect is preferable in which the sample addition pad has not only a function of receiving the added sample (test sample) containing a test substance, but also a function of filtering insoluble particles and the like in the sample. Examples of the material of the sample addition pad include materials having uniformity, such as cellulose filter paper, glass fiber, polyurethane, polyacetate, cellulose acetate, nylon, and cotton cloth. In addition, in order to prevent the test substance in the sample from being non-specifically adsorbed onto the material of the sample addition pad during the analysis and lowering the accuracy of the analysis, it is also possible to pretreating the material configuring the sample addition pad by a non-specific adsorption prevention treatment before use. In the present invention, the sample addition pad may also serve as the labeling substance-holding pad having the labeling substance-holding region described in (9-4).

### (9-6) Absorbent pad

In the present invention, an absorbent pad can be preferably used by being incorporated into the insoluble carrier. The absorbent pad is a site that physically absorbs the added test sample having moved chromatographically and absorbs and removes the labeling substance or the like not being captured by the reaction site of the chromatographic carrier. As the absorbent pad, a water-absorbent material such as cellulose filter paper, nonwoven fabric, or cellulose acetate is used. The speed at which the added test sample having chromatographically moved and reached the absorbent pad is to chromatographically move varies with the material, size, and the like of the absorbent pad. Therefore, by appropriately selecting the material, type, and the like of the absorbent pad, it is possible to set the speed suitable for testing the test substance.

### (10) Washing solution (second amplification solution)

In the present invention, the washing solution is a liquid having a function of washing off the labeling substance that remains (that is, non-specifically remains) in the insoluble carrier without having a specific binding reaction. The washing solution may be used after the pH thereof is adjusted to enhance the washing effect, or a washing solution to which a surfactant component or a polymer compound, such as a protein including BSA or the like, may be used. In the present invention, as the washing solution, it is preferable to use a liquid containing a reducing agent capable of reducing silver ions which will be described below. In that case, one of the two amplification solutions (described later) used will also serve as a washing solution.

Because the washing solution is developed while washing off the non-specifically remaining labeling substance, the labeled substance is incorporated into the developing washing solution. In order to enhance the washing effect, it is preferable that the washing solution to be used do not contain a labeling substance before being developed.

The washing solution is added to the insoluble carrier after the test sample (sample solution) is developed through the insoluble carrier, and washes off the labeling substance that remains in the insoluble carrier and is not the labeling substance having bound by a specific binding reaction. Feeding of the washing solution may be performed by a method of directly adding the washing solution to a sample dropping section after the sample solution is developed through the insoluble carrier, a method of preliminarily attaching an insoluble carrier for liquid feeding (washing solution addition pad) and an insoluble carrier for absorption (water-absorbent pad) to the insoluble carrier for feeding of the washing solution, adding the washing solution to the insoluble carrier for liquid feeding, feeding the washing solution toward the insoluble carrier for absorption, a method of preliminarily providing a washing solution addition site to the insoluble carrier, developing the sample solution, and then adding the washing solution to the washing solution addition site, or a method of developing the sample solution through the insoluble carrier and then attaching the insoluble carrier for liquid feeding and the insoluble carrier for absorption to the insoluble carrier for feeding of the washing solution.

In the present invention, the angle formed between the development direction of the test sample (sample solution) and the development direction of the washing solution is not particularly limited. The angle can be 0° to 180°, and is preferably 0° to 90°. The insoluble carrier for liquid feeding is not particularly limited as long as the washing solution can be added thereto. As the insoluble carrier for liquid feeding, a glass fiber pad, a cellulose membrane, a nitrocellulose membrane, or the like can be used. The insoluble carrier for absorption is not particularly limited as long as it is a substance capable of absorbing water. As the insoluble carrier for absorption, cellulose, nitrocellulose, glass fiber, a mixture of these, or the like can be used.

### (11) Immunoassay method

Hereinafter, a sandwich method, which is a specific embodiment of immunochromatography, will be described.

The sandwich method is not particularly limited. In this method, for example, a test substance can be analyzed by the following procedure. First, a first binding substance (for example, a first antibody) and a second binding substance (for example, a second antibody) having specificity for a test substance (antigen) are prepared in advance by the method described above. In addition, a labeling substance is modified in advance with the first binding substance. The second binding substance is immobilized on an appropriate insoluble carrier (for example, a nitrocellulose membrane, a glass fiber membrane, a nylon membrane, a cellulose membrane, or the like) to form a test region of a labeling substance capture region, and brought into contact with a test sample (or the extract thereof) that is likely to contain a test substance (antigen). In a case where the test substance is in the test sample, the test substance binds to the second binding substance (for example, an antigen-antibody reaction with the second antibody). In a case where an excess of labeling substance modified with the first binding substance is brought into contact with the test sample simultaneously with or after the binding of the test substance to the second binding substance, provided that the test substance is in the test sample, a complex consisting of the immobilized second binding substance, the test substance (antigen), and the labeling substance modified with the first binding substance is formed.

In the sandwich method, after the reaction among the immobilized second binding substance, the test substance (antigen), and the first binding substance modifying the labeling substance is terminated, the labeling substance not forming the immune complex is removed, and then, for example, the labeling substance capture region of the insoluble carrier is observed as it is to detect or quantify the labeling substance, which makes it possible to determine the presence or absence of the test substance in the test sample or to measure the amount of the test substance. In the present invention, for example, a reducing agent and a silver ion-containing compound are supplied to amplify and detect the signal from the labeling substance forming the aforementioned complex.

### (12) Amplification solution

The amplification solution used in the present invention is a liquid containing an amplification reagent. The amplification reagent is a reagent capable of causing signal amplification by catalytically reacting by the action of the labeling substance or test substance and generating a colored compound or inducing luminescence or the like. The amplification reagent can be used in the form of a solution containing a reagent, that is, as an amplification solution. Examples thereof include a silver ion solution that causes precipitation of metallic silver on a metal label by physical development, a solution of a phenylenediamine compound and a naphthol compound that turns into a colorant by the action of a peroxidase label and hydrogen peroxide, and the like.

Specifically, a so-called developer described in general books in the field of photographic chemistry (for example, "Revised Fundamentals of Photoengineering -Silver Halide Photography-" (edited by The Society of Photography and Imaging of Japan, CORONA PUBLISHING CO., LTD.), "Chemistry of Photography" (Akira Sasai, Shashin Kogyo Shuppansha), "Latest Formulation Handbook" (Shinichi Kikuchi et al., Amico Publishing Co., Ltd.)) can be used as the amplification solution containing an amplification reagent. That is, any developer can be used as an amplification solution without particular limitation, as long as the developer is a so-called physical developer that contains silver ions reduced around colloidal metal or the like which may be the nucleus of development.

In the present invention, two amplification reagents can be used. As for the two amplification reagents used for amplifying signals of the labeling substance captured in the labeling substance capture region, it is preferable that a first amplification reagent be preliminarily incorporated into a first amplification solution and a second amplification reagent be preliminarily incorporated into a second amplification solution, and that the second amplification solution and the first amplification solution be sequentially added to perform amplification.

Specifically, for example, as the amplification solution, it is possible to use the second amplification solution containing a reducing agent capable of reducing silver ions and the first amplification solution containing a silver-containing compound in combination.

Hereinafter, the reducing agent capable of reducing silver ions contained in the second amplification solution, the silver-containing compound contained in the first amplification solution, and the like will be described.

### (12-1) Silver-containing compound

As the silver-containing compound, it is possible to use a silver ion-containing compound, for example, an organic silver salt, an inorganic silver salt, or a silver complex. The silver-containing compound is preferably a silver ion-containing compound having high solubility in a solvent such as water, and examples thereof include silver nitrate, silver acetate, silver lactate, silver butyrate, silver thiosulfate, and the like. Among these, silver nitrate is particularly preferable. The silver complex is preferably a silver complex coordinated to a ligand having a water-soluble group such as a hydroxyl group or a sulfone group, and examples thereof include hydroxythioether silver and the like.

Generally, the content of silver contained in the inorganic silver salt or the silver complex is 0.001 mol/m² to 0.2 mol/m², and preferably 0.01 mol/m² to 0.05 mol/m².

### (12-2) Reducing agent capable of reducing silver ions

As the reducing agent capable of reducing silver ions, any inorganic or organic material or a mixture thereof can be used as long as these can reduce silver ions into silver.

Preferred examples of the inorganic reducing agent include a reducing metal salt and a reducing metal complex salt that can undergo valence change by metal ions such as Fe²⁺, V²⁺, and Ti³⁺. In a case where an inorganic reducing agent is used, oxidized ions need to be removed or made harmless by the formation of a complex or reduction. For example, in a system in which Fe²⁺ is used as a reducing agent, by using citric acid or EDTA, it is possible to make Fe³⁺ harmless by forming a complex of Fe³⁺ which is an oxide. In this system, an inorganic reducing agent is preferably used, and a metal salt of Fe²⁺ is more preferably used.

It is also possible to use developing agents used as photosensitive materials for wet silver halide photography (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid (or derivatives thereof), and leuco colorants) and other materials known to those skilled in the related art, such as the materials described in US6020117A.

As the reducing agent, an ascorbic acid reducing agent is also preferable. Useful ascorbic acid reducing agents include ascorbic acid, analogs and isomers of ascorbic acid, and derivatives thereof. Preferred examples thereof include D- or L-ascorbic acid and sugar derivatives thereof (for example, γ-lactoascorbic acid, glucoascorbic acid, fucoascorbic acid, glucoheptoascorbic acid, and maltoascorbic acid), sodium salt of ascorbic acid, potassium salt of ascorbic acid, isoascorbic acid (or L-erythroascorbic acid), salts thereof (for example, an alkali metal salt, ammonium salt, or a salt known in the technical field of related art), enediol-type ascorbic acid, enaminol-type ascorbic acid, thioenoyl-type ascorbic acid, and the like. Particularly, D-, L-, or D,L-ascorbic acid (and an alkali metal salt thereof) or isoascorbic acid (or an alkali metal salt thereof) is preferable, and a sodium salt is preferable as a salt thereof. As necessary, a mixture of these reducing agents can be used.

### (13) Other aids

In some cases, the amplification solution contains, as other aids, a buffer, a preservative (for example, an antioxidant or an organic stabilizer), and a rate control agent. As the buffering, for example, it is possible to use acetic acid, citric acid, sodium hydroxide, salts thereof, a buffer using tris(hydroxymethyl)aminomethane, and other buffers used in a general chemical experiment. Appropriately using these buffers makes it possible to adjust the pH to a level optimal for the amplification solution. In addition, as an antifogging agent, an alkylamine can be used as an additive, which is particularly preferably dodecylamine. Furthermore, in order to improve the solubility of these additives, a surfactant can be used, which is particularly preferably C₉H₁₉-C₆H₄-O-(CH₂CH₂O)₅₀H.

It is preferable that the amplification reagent be dispensed to the immunochromatography kit by a method of dispensing a reducing agent solution as the second amplification solution to a pad for feeding the reducing agent solution, feeding the reducing agent solution into the labeling substance capture region, and dispensing a silver ion solution as the first amplification solution to a region including the labeling substance capture region from above the insoluble carrier such that the silver ion solution infiltrates the insoluble carrier in the thickness direction.

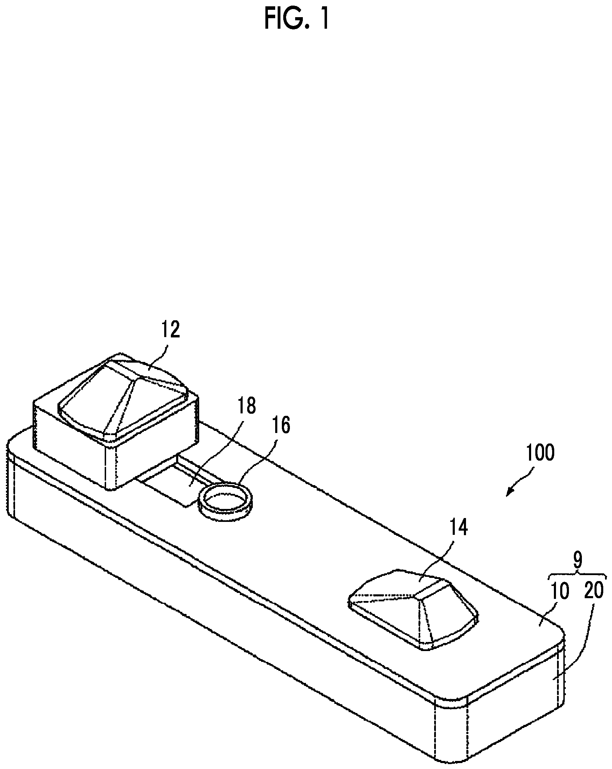

Examples of methods of preparing an immunochromatography kit containing two built-in amplification reagents include a method of arranging, as pots (containers) containing the amplification reagents, a second amplification solution storage pot (a second container) containing a reducing agent solution and a first amplification solution storage pot (a first container) on a portion above the site to which the amplification reagents are to be dispensed. For example, an aspect may be adopted in which the second amplification solution storage pot (the second container) containing a reducing agent solution is placed above a pad for feeding the reducing agent solution, and the first amplification solution storage pot (the first container) containing a silver ion solution (first amplification solution) is placed immediately above a silver ion solution filling hole. Regarding a more detailed addition method, arranging the pots as in the device shown in Fig. 1 of JP5276568B and pressing the pots makes it possible to cause the amplification solutions to flow and to be dispensed to predetermined sites.

The amplification reagents can also be dispensed to the immunochromatography kit by a method of installing the second amplification solution storage pot containing a reducing agent solution in a portion of the sample addition pad, the portion corresponding to an end of the insoluble carrier opposite to the labeling substance capture region, and supplying the reducing agent solution into the insoluble carrier such that the reducing agent solution is developed after the test sample in the same direction as the development direction of the test sample. For example, an aspect may be adopted in which after the labeling substance capture region is filled with the reducing agent solution, a silver ion solution as the first amplification solution is dispensed to the labeling substance capture region from above the insoluble carrier such that the silver ion solution infiltrates the insoluble carrier in the thickness direction. Regarding a more detailed addition method, arranging projection-like deformable portions as in the device shown in Figs. 1 to 8 of JP6570652B and pressing such portions makes it possible to cause the amplification solutions to flow and to be dispensed to predetermined positions.

### (14) Immunochromatography kit

The detection method according to the embodiment of the present invention can be performed using an immunochromatography kit including a labeling substance that is modified with a first binding substance capable of binding to a test substance and an insoluble carrier including a second binding substance that is capable of binding to the test substance or a substance that exhibits binding properties to the first binding substance capable of binding to the test substance. In this case, the labeling substance modified with the first binding substance capable of binding to the test substance may be provided in advance on the insoluble carrier of the immunochromatography kit. Alternatively, the labeling substance modified with the first binding substance capable of binding to the test substance may be provided separately from the insoluble carrier. In this case, the test can be performed by a method of mixing the labeling substance provided separately from the insoluble carrier with the test sample and then developing the test sample on the insoluble carrier. The immunochromatography kit according to the embodiment of the present invention comprises a washing solution and amplification solutions. Preferably, the immunochromatography kit can include a first amplification solution that contains a silver-containing compound, and a second amplification solution that also functions as a washing solution containing a reducing agent capable of reducing silver ions. As examples of the materials configuring the immunochromatography kit and preferred ranges thereof, the examples and ranges described above regarding immunochromatography and the like can be used.

The present invention will be more specifically described based on the following examples, but the present invention is not limited to the examples.

### Examples

The immunochromatography kits of examples are an immunochromatography kit for SARS-CoV-2 antigen detection for detecting an NP antigen.

### Experiment A

### <1> Preparation of anti-NP monoclonal antibody

### (1) Mouse immunization and hybridoma cell preparation

A purified antigenic protein (300 µg, the protein having the amino acid sequence described in SEQ ID NO: 1) was emulsified by being mixed with Freund's adjuvant, and administered to a BALB/c mouse multiple times by subcutaneous injection (300 µg of the antigenic protein was administered per dose) such that the mouse was immunized. After one month, B cells were collected from the immunized mouse and fused with myeloma cells by using a polyethylene glycol solution. The fused hybridoma cells were seeded on a 96-well microplate together with a selective medium.

### (2) Screening and cloning of hybridoma cells

Next, by the enzyme-linked immunosorbent assay (ELISA) and western blotting, the reactivity of the monoclonal antibody produced by each hybridoma with respect to SARS-CoV-2NP was examined. In ELISA, first, 5 µg of the His-tagged recombinant SARS-CoV-2 NP protein was immobilized on an ELISA plate. After blocking the plate, 100 µL of the culture supernatant of the hybridoma was added to the plate for reaction, and then a peroxidase-labeled goat anti-mouse immunoglobulin antibody was added thereto for reaction. Then, a peroxidase substrate solution was added thereto to develop color, and the absorbance thereof was measured. In the western blotting, first, 1 µg of the His-tagged recombinant SARS-CoV-2 NP protein was mixed with a 2X SDS sample buffer solution (125 mM Tris-HCl, 4% SDS, 20% glycerol, 0.01% bromophenol blue, 10% 2-mercaptoethanol), thermally treated, heat-treated, loaded on a polyacrylamide gel and subjected to electrophoresis. The gel after electrophoresis was transferred to a PVDF membrane. The membrane was blocked with a 2% skim milk solution, 5 mL of the culture supernatant of the hybridoma was then added thereto for reaction, and the cells were reacted with a peroxidase-labeled anti-mouse immunoglobulin antibody. Thereafter, a peroxidase substrate solution was added thereto to induce luminescence, and detection was performed. The hybridoma identified through these screening steps was screened, thereby obtaining hybriddoma cells producing a monoclonal antibody showing high reactivity to the antigenic protein.

### (3) Preparation of monoclonal antibody

Each hybridoma was cultured in a serum-free and protein-free culture solution, and the culture supernatant was collected. From the collected culture supernatant, a monoclonal antibody produced by each hybridoma cell was purified using a protein A column. First, 120 mL of the culture supernatant was 2X diluted with an equilibration·washing buffer solution (1.5 mol/L Glycine, 3 mol/L NaCl, pH 8.9) and added to a protein A column (volume: 1.04 mL) equilibrated with an equilibration·washing buffer solution such that the antibody was captured in the protein A carrier, and the components nonspecifically adsorbed onto the carrier was washed away with 10 mL of an equilibration·washing buffer solution. Next, the antibody adsorbed onto the column was eluted with 10 mL of an elution buffer solution (100 mmol/L sodium citrate, pH 3.0). The collected eluent was concentrated using AMICON ULTRA (Merck Millipore). The concentration of the obtained purified antibody was quantified by a UV absorption method at 280 nm.

### (4) Determination of subclass

Subclasses were identified using the ISO-GOLD Rapid Isotyping Kit. The purified antibodies were 100X diluted with a Sample Diluent Buffer. 150 µL of this solution was added to a lateral flow kit and left to stand for 5 minutes. The detected line was read, and the subclass was identified. A plurality of antibodies was prepared.

Through the above process, four types of antibodies, IgG2b (No1), IgG2b (No2), IgG1 (No1), and IgG1 (No2), were obtained.

### <2> Preparation of immunochromatography kit

### <Example 1>

### (2-1) Preparation of anti-NP antibody-modified colloidal gold as labeling substance modified with first substance capable of binding to test substance

A 50 mmol/L KH₂PO₄ buffer (1 mL, pH 8.0) was added to 9 mL of a solution containing a colloidal gold having a diameter of 50 nm (product number: EM. GC50, manufactured by BBI Solutions.) for pH adjustment, and then added to 1 mL of a 20 µg/mL anti-NP monoclonal antibody (antibody that belongs to the subclass iGg2b (No1))-containing solution, followed by stirring for 10 minutes. The solution was then left to stand for 10 minutes, and then 550 µL of an aqueous solution containing 1% by mass of polyethylene glycol (PEG; weight-average molecular weight (Mw.): 20,000, product number: 168-11285, manufactured by FUJIFILM Wako Pure Chemical Corporation), followed by stirring for 10 minutes. Thereafter, an aqueous solution of 10% by mass bovine serum albumin (BSA; Fraction V, product number: A-7906, manufactured by Sigma-Aldrich Corporation) was added thereto, followed by stirring for 10 minutes. This solution was centrifuged for 30 minutes under the conditions of 8000xg and 4°C by using a centrifugal separator (himac CF16RX, manufactured by Hitachi, Ltd.). The supernatant solution was removed leaving 1 mL at the bottom of the container, and the colloidal gold contained in the 1 mL of the solution remaining at the bottom of the container was redispersed by an ultrasonic washer. Then, the solution was dispersed in 20 mL of a colloidal gold preservation solution (20 mmol/L Tris-HCl (tris-hydrochloric acid) buffer (pH 8.2), 0.05% PEG (Mw. 20,000), 150 mmol/L NaCl, 1% BSA) and centrifuged again under the same conditions as above by using the same centrifugal separator, the supernatant solution was removed, subjected to ultrasonic dispersion, and then dispersed in a colloidal gold preservation solution, thereby obtaining an antibody-modified colloidal gold (50 nm) solution.

### (2-2) Preparation of anti-NP antibody-modified colloidal gold-holding pad as label holding pad

The anti-NP antibody-modified colloidal gold prepared in (2-1) was diluted with water such that the Tris-HCl buffer (pH 8.2) concentration was 20 mmol/L, the PEG (Mw. 20,000) concentration was 0.05% by mass, the sucrose concentration was 5% by mass, and the optical density of the colloidal gold at 520 nm was 0.1 in a case where optical path length was set to 10 mm, thereby obtaining a colloidal gold coating liquid. Each glass fiber pad (Glass Fiber Conjugate Pad, manufactured by Merck Millipore) cut in 5 mm × 300 mm was uniformly coated with 1 mL of this coating liquid and dried under reduced pressure for 24 hours, thereby obtaining an anti-NP antibody-modified colloidal gold-holding pad.

### (2-3) Preparation of chromatographic carrier

As an insoluble carrier, a nitrocellulose membrane (with a plastic lining, HiFlow Plus HF135 (capillary flow rate = 135 sec/cm), manufactured by Merck Millipore) cut in 60 mm × 300 mm was used. On the membrane, as capture regions, the following three a test region, a confirmation region, and an amplification index region, were formed by the following method, thereby preparing a chromatographic carrier.

An anti-NP monoclonal antibody (antibody that belongs to the subclass IgG2b (No2)) solution prepared to have a concentration of 1.5 mg/mL was linearly applied to a position 15 mm distant from the downstream short side between the 60 mm long short sides of the nitrocellulose membrane, thereby forming a test region. Furthermore, an anti-mouse IgG antibody solution prepared to have a concentration of 0.5 mg/mL was linearly applied to a position 11 mm distant from the downstream short side between the 60 mm long short sides, thereby forming a confirmation region. In addition, a bromocresol green (manufactured by FUJIFILM Wako Pure Chemical Corporation) prepared to have a concentration of 30 mmol/L was linearly applied to a position 9 mm distant from the downstream short side between the 60 mm short sides, thereby forming an amplification index region. After the above solutions are applied to the respective positions, the nitrocellulose membrane was dried at 50°C for 30 minutes in a hot air drier. After the drying ended, the nitrocellulose membrane dried as above was immersed in a pad containing 500 mL of a blocking solution (50 mmol/L borate buffer (pH 8.5) containing 0.5% by mass casein (derived from milk, product number 030-01505, manufactured by FUJIFILM Wako Pure Chemical Corporation)), and left to stand as it was for 30 minutes. Then, the nitrocellulose membrane was taken out, immersed in 500 mL of a washing·stabilizing solution (50 mmol/L Tris-HCl (pH 7.5) buffer containing 0.5% by mass sucrose and 0.05% by mass sodium cholate) prepared in another pad, and left to stand as it was for 30 minutes. Thereafter, the nitrocellulose membrane was taken out of the solution and dried in an environment of 25°C for 24 hours. The portion where the anti-NP antibody is immobilized corresponds to the test region containing a second substance binding to a test substance, the portion where the anti-mouse IgG antibody is immobilized corresponds to the confirmation region containing a substance capable of binding to a first substance, and the portion where the bromocresol green is immobilized corresponds to an amplification index region containing a substance reacting with a first amplification solution. These three regions are collectively called a capture region.

### (2-4) Preparation of test strip

The chromatographic carrier prepared in (2-3) was attached to a back pressure-sensitive adhesive sheet (60 mm × 300 mm (manufactured by Adhesives Research)). Then, a double-sided tape (Nitto Denko Corporation.) having a width of 3 mm was fixed to a position 26 mm distant from the downstream short side between the short sides of the chromatographic carrier. Thereafter, the downstream end of the double-sided tape and the downstream end of a glass fiber pad cut in 8 mm × 300 mm (Glass Fiber Conjugate Pad, manufactured by Merck Millipore) were made overlapped with each other, and the colloidal gold-holding pad was fixed to the chromatographic carrier. A liquid feeding pad (a glass fiber pad cut in 25 mm × 300 mm (Glass Fiber Conjugate Pad, manufactured by Merck Millipore)) was attached to the upstream side of the chromatographic carrier such that the liquid feeding pad and the chromatographic carrier overlapped with each other by 7 mm. With a guillotine cutter (CM 4000, manufactured by NIPPN TechnoCluster Inc.), the member prepared as above was cut along a direction parallel to a direction perpendicular to the 300 mm long side such that the cut piece had a width of 5 mm. In this way, 60 test strips (without an absorbent pad) were prepared.

### (2-5) Preparation of amplification solution

### (2-5-1) Preparation of amplification solution (reducing agent solution) to be sealed in second pot

A 1 mol/L aqueous iron nitrate solution (23.6 mL), which was prepared by dissolving iron (III) nitrate nonahydrate (manufactured by FUJIFILM Wako Pure Chemical Corporation, 095-00995) in water, and 13.1 g of citric acid (manufactured by FUJIFILM Wako Pure Chemical Corporation, 038-06925) was dissolved in 290 g of water. After all of the substances were dissolved, 36 mL of a nitric acid (10% by weight) solution was added thereto with stirring with a stirrer, and 60.8 g of ammonium iron (II) sulfate hexahydrate (manufactured by FUJIFILM Wako Pure Chemical Corporation, 091-00855) was added thereto. The solution prepared in this way was used as a reducing agent solution which is a second amplification solution to be sealed in a second pot.

### (2-5-2) Preparation of amplification solution (silver ion solution) to be sealed in first pot

A silver nitrate solution (8 mL, containing 10 g of silver nitrate) and 24 mL of a 1 mol/L aqueous iron nitrate solution were added to 66 g of water. This solution was mixed with a solution which was prepared in advance by dissolving 5.9 mL of nitric acid (10% by weight), 0.1 g of dodecylamine (manufactured by FUJIFILM Wako Pure Chemical Corporation, 123-00246), and 0.1 g of a surfactant C₁₂H₂₅-C₆H₄-O-(CH₂CH₂O)₅₀H in 47.6 g of water, and the obtained solution was used as a silver ion solution which is a first amplification solution to be sealed in a first pot.

### (2-6) Preparation of absorbent pad

Sixty glass fiber pads (glass filter paper, manufactured by ADVANTEC CO., LTD.) cut in 12 mm × 10 mm were prepared and used as absorbent pads.

### (2-7) Preparation of parts for immunochromatography kit

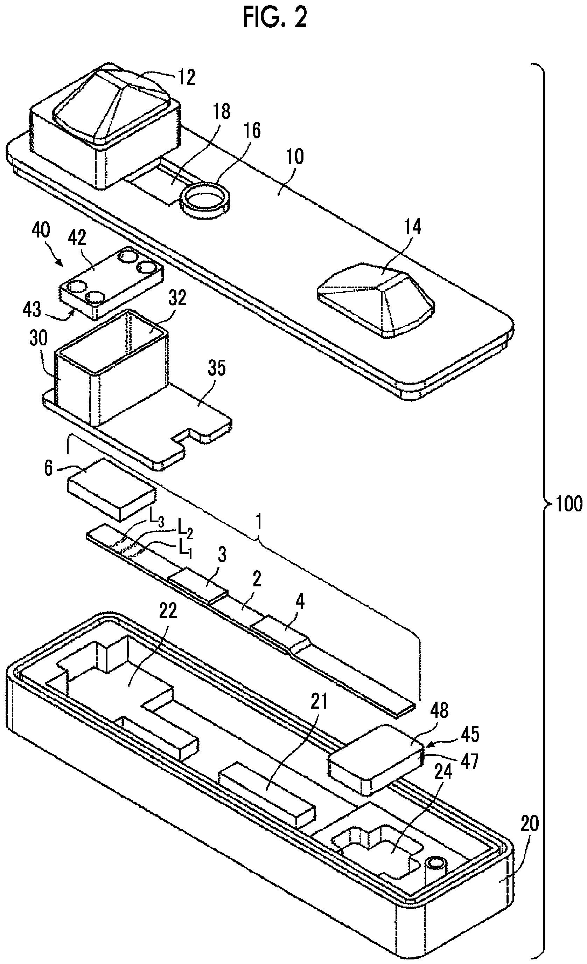

A lower case 20, an upper case 10, an intermediate member 30, a first pot 40, and a second pot 45 constituting an immunochromatography kit 100 shown in Figs. 1 and 2 were prepared by injection molding by using polypropylene as a material. The upper case was prepared by injection molding by using polypropylene containing 50% by mass of TAFCELENE (registered trademark), which is an olefin-based elastomer manufactured by Sumitomo Chemical Co., Ltd., as a material. The upper case 10 comprises two deformable sites (a first projection-like deformable portion and a second projection-like deformable portion). These two deformable portions do not have a portion to be separated from the upper case 10, and made by injection molding as a part of the upper case 10 at all boundary portions.

In examples, the upper case was configured such that a first projection-like deformable portion 12 shown in Figs. 1 and 2 have two projections and a second projection-like deformable portion 14 has one projection.

### (2-8-1) Preparation of immunochromatography kit of Example 1

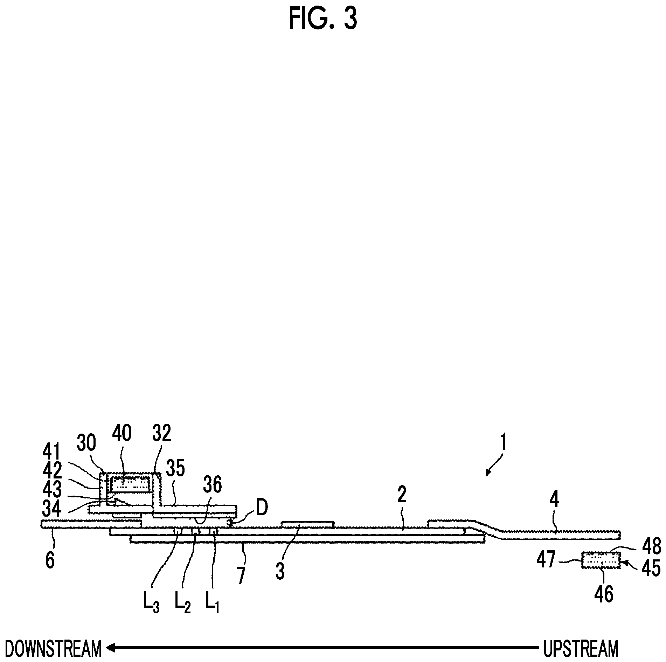

The lower case 20, a test strip 1 prepared in (1-4), and an absorbent pad 6 prepared in (1-6) were fixed as shown in Figs. 1 and 2. Then, as shown in Fig. 3, the first pot 40 was filled with a first amplification solution 41 to be sealed in the first pot 40 prepared in (1-5-2), and the second pot 45 was filled with a second amplification solution 46 to be sealed in the second pot 45 prepared in (1-5-1), the pot 45 was sealed with aluminum foil as a sheet member 48, and the pot 40 was sealed with aluminum foil as a sheet member 43. Furthermore, as shown in Figs. 1 and 2, the second pot 45 was mounted on the lower case 20 with the sheet member 48 facing up, and the first pot 40 was mounted on the intermediate member 30 with the sheet member 43 facing down. Thereafter, in a state where the upper case 10 and the lower case 20 were fitted such that the outer periphery thereof came into contact with each other, the contact portions between the upper case and the lower case were joined by ultrasonic welding. At this time, the welded sites were checked to make sure that all the sites are uniformly welded in a sealed state. An immunochromatography kit was prepared in this manner.

### <Example 2>

A gold chromatography kit of Example 2 was prepared in the same manner as in Example 1, except that in preparing the anti-NP antibody-modified colloidal gold described in (2-1) of Example 1, an antibody that belongs to the subclass IgG2b (No2) was used instead of the antibody that belongs to the subclass IgG2b (No1), and that in preparing the chromatographic carrier described in (2-3) of Example 1, an antibody that belongs to the subclass IgG2b (No1) was used instead of the antibody that belongs to the subclass IgG2b (No2).

### <Example 3>

A gold chromatography kit of Example 3 was prepared in the same manner as in Example 1, except that in preparing the chromatographic carrier described in (2-3) of Example 1, an antibody that belongs to the subclass IgG1 (No2) was used instead of the antibody that belongs to the subclass IgG2b (No2).

### <Comparative Example 1>

A gold chromatography kit of Comparative Example 1 was prepared in the same manner as in Example 1, except that in preparing the anti-NP antibody-modified colloidal gold described in (2-1) of Example 1, an antibody that belongs to the subclass IgG1 (No1)was used instead of the antibody that belongs to the subclass IgG2b (No1), and that in preparing the chromatographic carrier described in (2-3), an antibody that belongs to the subclass IgG1 (No2) was used instead of the antibody that belongs to the subclass IgG2b (No2).

### <Comparative Example 2>

A gold chromatography kit of Comparative Example 2 was prepared in the same manner as in Example 1, except that in preparing the anti-NP antibody-modified colloidal gold described in (2-1) of Example 1, an antibody that belongs to the subclass IgG1 (No2) was used instead of the antibody that belongs to the subclass IgG2b (No1), and that in preparing the chromatographic carrier described in (2-3), an antibody that belongs to the subclass IgG1 (No1) was used instead of the antibody that belongs to the subclass IgG2b (No2).

### <3> Evaluation of detection sensitivity

### (3-1) Evaluation solution

Tween (registered trademark) 80 (4 g) was added to 3,280 mL of ultrapure water and dissolved. Trishydroxydimethylmethane (120 g), 4 g of casein, and 60 g of EDTA·Na (sodium ethylenediaminetetraacetate) were added thereto. The pH was adjusted to 7.7, and then the volume was increased to 4 L.

### (3-2) Preparation of sample solution

The SARS-CoV-2 recombinant NP prepared in (1) described above was added to the evaluation solution prepared as described above, thereby preparing sample solutions of each concentration.

### (3-3) Test using immunochromatographic kits of examples and comparative examples

The sample (24 µL) prepared as above was dispensed to the kit.

Immediately after the dispensing of the sample, a second projection-like deformable portion 14 pressed down, such that the aluminum foil as the sheet member 48 sealing a second amplification solution 46 sealed in the second pot 45 is broken, and that a liquid feeding pad 4 is immersed in the second pot 45. In this way, the second amplification solution 46 was supplied to the insoluble carrier 2 by using capillary action.

After an amplification index region L3 turns orange from green, a first projection-like deformable portion 12 was pressed down, such that first pot 40 moved to a breaking portion 34 of the first pot housing portion 32 of the intermediate member 30. In this way, the aluminum foil as the sheet member 43 sealing the first pot 40 was broken by pressing the breaking portion 34, the silver ion solution which is a first amplification solution 41 was supplied to the insoluble carrier 2 from the opening portion of the intermediate member 30, and a silver amplification reaction was performed. The silver amplification reaction is completed in several tens of seconds.

After the completion of the silver amplification reaction, the kit was observed to determine whether the sample is positive or negative. For the determination, a capture amount was determined by imaging the extent of development of black of the anti-NP antibody coating line that increases or decreases in proportion to the NP amount by using LAS 4000 (manufactured by GE Healthcare Technologies Inc.), and calculating the signal.

### Evaluation was performed based on the following standard.

A: The signal concentration exceeds 0.1, and the presence or absence of the sample can be sufficiently confirmed.
B: The signal concentration is 0.05 to 0.1, the increase in concentration is detectable, and the presence or absence of the sample is confirmed.
C: The signal concentration is 0.03 to 0.05, and the sample is barely detected.
D: Signal concentration is 0.03 or less. The sample is absent or undetectable.

The results are shown in Table 1.

**[Table 1]**

| | Colloidal gold antibody | Membrane antibody | Concentration of SARS-CoV- 2 recombinant NP in evaluation solution (pg/ml) | | | | | |
|---|---|---|---|---|---|---|---|---|
| | | | 800 | 100 | 25 | 12.5 | 6.3 | 0 |
| Example 1 | IgG2b (No1) | IgG2b (No2) | A | A | B | C | C | D |
| Example 2 | IgG2b (No2) | IgG2b (No1) | A | A | B | C | C | D |
| Example 3 | IgG2b (No1) | IgG1 (No2) | A | A | B | C | C | D |
| Comparative Example 1 | IgG1 (No1) | IgG1 (No2) | C | D | D | D | D | D |
| Comparative Example 2 | IgG1 (No2) | IgG1 (No1) | C | D | D | D | D | D |

It has been found that the detection sensitivity is high in a case where at least one type of IgG2b antibody is used as the anti-NP monoclonal antibody of the immunochromatography kit.

The minimum detection sensitivity described in the document attached to the SARS-CoV-2 antigen detection reagent "ESPLINE SARS-CoV-2" manufactured by FUJIREBIO Inc. is 25 pg/mL. It has been found that the sensitivity of the immunochromatography kits of examples is likely to be higher than the detection sensitivity.

### Experiment B

### <1> Fragmentation of antibody

Disodium phosphate was dissolved in pure water, and the pH was adjusted to 7.8 with 1 mol/L hydrochloric acid, thereby preparing a digestion solution. The buffer solution of the antibody was replaced with the digestion solution by using a desalting column (Zebatm Spin Desalting Columns, 7K MWCO). Endoproteinase Glu-C (V8 Protease) (manufactured by Sigma-Aldrich Co. LLC or FUJIFILM Wako Pure Chemical Corporation) was weighed (2.0 mg), and 300 µL of the digestion solution was added thereto, thereby preparing an enzyme solution. The enzyme solution (150 µL) was added to 2 mg of the antibody (IgG2b (No1)) and allowed to react at 37°C for 20 hours. After the reaction, ultrafiltration was performed using AMICON ULTRA (Merck Millipore), whole antibodies were removed using Monospin proA (manufactured by GL Sciences Inc.), and the fragmented antibody was purified.

### <2> Preparation of immunochromatography kit

### <Example 4>

### (2-1) Preparation of anti-NP fragmented antibody-modified colloidal gold as labeling substance modified with first substance capable of binding to test substance

A 50 mmol/L KH₂PO₄ buffer (1 mL, pH 8.0) was added to 9 mL of a solution containing a colloidal gold having a diameter of 50 nm (product number: EM. GC50, manufactured by BBI Solutions.) for pH adjustment, and then added to a 20 µg/mL fragmented anti-NP monoclonal antibody (1 mL of solution containing the antibody) prepared in (1), followed by stirring for 10 minutes. The solution was then left to stand for 10 minutes, and then 550 µL of an aqueous solution containing 1% by mass of polyethylene glycol (PEG; weight-average molecular weight (Mw.): 20,000, product number: 168-11285, manufactured by FUJIFILM Wako Pure Chemical Corporation) was added thereto, followed by stirring for 10 minutes. Thereafter, 1.1 mL of an aqueous solution of 10% by mass bovine serum albumin (BSA; Fraction V, product number: A-7906, manufactured by Sigma-Aldrich Corporation) was added thereto, followed by stirring for 10 minutes. This solution was centrifuged for 30 minutes under the conditions of 8000xg and 4°C by using a centrifugal separator (himac CF16RX, manufactured by Hitachi, Ltd.). The supernatant solution was removed leaving 1 mL at the bottom of the container, and the colloidal gold contained in the 1 mL of the solution remaining at the bottom of the container was redispersed by an ultrasonic washer. Then, the solution was dispersed in 20 mL of a colloidal gold preservation solution (20 mmol/L Tris-HCl (tris-hydrochloric acid) buffer (pH 8.2), 0.05% PEG (Mw. 20,000), 150 mmol/L NaCl, 1% BSA) and centrifuged again under the same conditions as above by using the same centrifugal separator, the supernatant solution was removed, subjected to ultrasonic dispersion, and then dispersed in a colloidal gold preservation solution, thereby obtaining a fragmented anti-NP antibody-modified colloidal gold (50 nm) solution.

### (2-2) Preparation of anti-NP antibody-modified colloidal gold-holding pad as label holding pad

The anti-NP antibody-modified colloidal gold prepared in (2-1) was diluted with water such that the Tris-HCl buffer (pH 8.2) concentration was 20 mmol/L, the PEG (Mw. 20,000) concentration was 0.05% by mass, the sucrose concentration was 5% by mass, and the optical density of the colloidal gold at 520 nm was 0.1 in a case where optical path length was set to 10 mm, thereby obtaining a colloidal gold coating liquid. Each glass fiber pad (Glass Fiber Conjugate Pad, manufactured by Merck Millipore) cut in 5 mm × 300 mm was uniformly coated with 1 mL of this coating liquid and dried under reduced pressure for 24 hours, thereby obtaining an anti-NP antibody-modified colloidal gold-holding pad.

### (2-3) Preparation of chromatographic carrier

As an insoluble carrier, a nitrocellulose membrane (with a plastic lining, HiFlow Plus H135 (capillary flow rate = 135 sec/cm), manufactured by Merck Millipore) cut in 60 mm × 300 mm was used. On the membrane, a test region, a confirmation region, and an amplification index region were formed by the following method, thereby preparing a chromatographic carrier.

An anti-NP monoclonal antibody (IgG1 (No2)) solution prepared to have a concentration of 1.5 mg/mL was linearly applied to a position 15 mm distant from the downstream short side between the 60 mm long short sides of the nitrocellulose membrane, thereby forming a test region. Furthermore, an anti-mouse IgG antibody solution prepared to have a concentration of 0.5 mg/mL was linearly applied to a position 11 mm distant from the downstream short side between the 60 mm long short sides, thereby forming a confirmation region. In addition, a bromocresol green (manufactured by FUJIFILM Wako Pure Chemical Corporation) prepared to have a concentration of 30 mmol/L was linearly applied to a position 9 mm distant from the downstream short side between the 60 mm short sides, thereby forming an amplification index region. After the above solutions are applied to the respective positions, the nitrocellulose membrane was dried at 50°C for 30 minutes in a hot air drier. After the drying ended, the nitrocellulose membrane dried as above was immersed in a pad containing 500 mL of a blocking solution (50 mmol/L borate buffer (pH 8.5) containing 0.5% by mass casein (derived from milk, product number 030-01505, manufactured by FUJIFILM Wako Pure Chemical Corporation)), and left to stand as it was for 30 minutes. Then, the nitrocellulose membrane was taken out, immersed in 500 mL of a washing·stabilizing solution (50 mmol/L Tris-HCl (pH 7.5) buffer containing 0.5% by mass sucrose and 0.05% by mass sodium cholate) prepared in another pad, and left to stand as it was for 30 minutes. Thereafter, the nitrocellulose membrane was taken out of the solution and dried in an environment of 25°C for 24 hours. The portion where the anti-NP antibody is immobilized corresponds to the test region containing a second substance binding to a test substance, the portion where the anti-mouse IgG antibody is immobilized corresponds to the confirmation region containing a substance capable of binding to a first substance, and the portion where the bromocresol green is immobilized corresponds to an amplification index region containing a substance reacting with a first amplification solution.

### (2-4) Preparation of test strip

The chromatographic carrier prepared in (2-3) was attached to a back pressure-sensitive adhesive sheet (60 mm × 300 mm (manufactured by Adhesives Research)). Then, a double-sided tape (Nitto Denko Corporation.) having a width of 3 mm was fixed to a position 26 mm distant from the downstream short side between the short sides of the chromatographic carrier. Thereafter, the downstream end of the double-sided tape and the downstream end of a glass fiber pad cut in 8 mm × 300 mm (Glass Fiber Conjugate Pad, manufactured by Merck Millipore) were made overlapped with each other, and the colloidal gold-holding pad prepared in (2-2) was fixed to the chromatographic carrier. A liquid feeding pad (a glass fiber pad cut in 25 mm × 300 mm (Glass Fiber Conjugate Pad, manufactured by Merck Millipore)) was attached to the upstream side of the chromatographic carrier such that the liquid feeding pad and the chromatographic carrier overlapped with each other by 7 mm. With a guillotine cutter (CM 4000, manufactured by NIPPN TechnoCluster Inc.), the member prepared as above was cut along a direction parallel to a direction perpendicular to the 300 mm long side such that the cut piece had a width of 5 mm. In this way, 60 test strips (without an absorbent pad) were prepared.

### (2-5) Preparation of amplification solution

### (2-5-1) Preparation of amplification solution (reducing agent solution) to be sealed in second pot

A 1 mol/L aqueous iron nitrate solution (23.6 mL), which was prepared by dissolving iron (III) nitrate nonahydrate (manufactured by FUJIFILM Wako Pure Chemical Corporation, 095-00995) in water, and 13.1 g of citric acid (manufactured by FUJIFILM Wako Pure Chemical Corporation, 038-06925) was dissolved in 290 g of water. After all of the substances were dissolved, 36 mL of a nitric acid (10% by weight) solution was added thereto with stirring with a stirrer, and 60.8 g of ammonium iron (II) sulfate hexahydrate (manufactured by FUJIFILM Wako Pure Chemical Corporation, 091-00855) was added thereto. The solution prepared in this way was used as a reducing agent solution which is a second amplification solution to be sealed in a second pot.

### (2-5-2) Preparation of amplification solution (silver ion solution) to be sealed in first pot

A silver nitrate solution (8 mL, containing 10 g of silver nitrate) and 24 mL of a 1 mol/L aqueous iron nitrate solution were added to 66 g of water. This solution was mixed with a solution which was prepared in advance by dissolving 5.9 mL of nitric acid (10% by weight), 0.1 g of dodecylamine (manufactured by FUJIFILM Wako Pure Chemical Corporation, 123-00246), and 0.1 g of a surfactant C₁₂H₂₅-C₆H₄-O-(CH₂CH₂O)₅₀H in 47.6 g of water, and the obtained solution was used as a silver ion solution which is a first amplification solution to be sealed in a first pot.

### (2-6) Preparation of absorbent pad

Sixty glass fiber pads (glass filter paper, manufactured by ADVANTEC CO., LTD.) cut in 12 mm × 10 mm were prepared and used as absorbent pads.

### (2-7) Preparation of parts for immunochromatography kit

A lower case 20, an upper case 10, an intermediate member 30, a first pot 40, and a second pot 45 constituting an immunochromatography kit 100 shown in Figs. 1 and 2 were prepared by injection molding by using polypropylene as a material. The upper case was prepared by injection molding by using polypropylene containing 50% by mass of TAFCELENE (registered trademark), which is an olefin-based elastomer manufactured by Sumitomo Chemical Co., Ltd., as a material. The upper case 10 comprises two deformable sites (a first projection-like deformable portion and a second projection-like deformable portion). These two deformable portions do not have a portion to be separated from the upper case 10, and made by injection molding as a part of the upper case 10 at all boundary portions.

In examples, the upper case was configured such that a first projection-like deformable portion 12 shown in Figs. 1 and 2 have two projections and a second projection-like deformable portion 14 has one projection.

### (2-8) Preparation of immunochromatography kit of examples

The lower case 20, the test strip 1 prepared in (2-4), and the absorbent pad 6 prepared in (2-6) were fixed as shown in Figs. 1 and 2. Then, as shown in Fig. 3, the first pot 40 was filled with a first amplification solution 41 to be sealed in the first pot 40 prepared in (1-5-2), and the second pot 45 was filled with a second amplification solution 46 to be sealed in the second pot 45 prepared in (1-5-1), the second pot 45 was sealed with aluminum foil as a sheet member 48, and the first pot 40 was sealed with aluminum foil as a sheet member 43. Furthermore, as shown in Figs. 1 and 2, the second pot 45 was mounted on the lower case 20 with the sheet member 48 facing up, and the first pot 40 was mounted on the intermediate member 30 with the sheet member 43 facing down. Thereafter, in a state where the upper case 10 and the lower case 20 were fitted such that the outer periphery thereof came into contact with each other, the contact portions between the upper case and the lower case were joined by ultrasonic welding. At this time, the welded sites were checked to make sure that all the sites are uniformly welded in a sealed state. An immunochromatography kit was prepared in this manner.

### <Example 5>

An immunochromatographic kit of Example 5 was prepared in the same manner as in Example 4, except that the antibody was changed to the antibody shown in the following Table 2 in Example 4.

### <3> Evaluation of detection sensitivity

### (3-1) Evaluation solution

Tween (registered trademark) 80 (4 g) was added to 3,280 mL of ultrapure water and dissolved. Trishydroxydimethylmethane (120 g), 4 g of casein, and 60 g of EDTA·Na were added thereto. The pH was adjusted to 7.7, and then the volume was increased to 4 L.

### (3-2) Preparation of sample solution

SARS-CoV-2 recombinant NP was added to the evaluation solution, thereby preparing samples of each concentration.

The sample (24 µL) prepared as above was dispensed to the kit.

Immediately after the dispensing of the sample, a second projection-like deformable portion 14 pressed down, such that the aluminum foil as the sheet member 48 sealing a second amplification solution 46 sealed in the second pot 45 is broken, and that a liquid feeding pad 4 is immersed in the second pot 45. In this way, the second amplification solution 46 was supplied to the insoluble carrier 2 by using capillary action.

After an amplification index region L3 turns orange from green, a first projection-like deformable portion 12 was pressed down, such that first pot 40 moved to a breaking portion 34 of the first pot housing portion 32 of the intermediate member 30. In this way, the aluminum foil as the sheet member 43 sealing the first pot 40 was broken by pressing the breaking portion 34, the silver ion solution which is a first amplification solution 41 was supplied to the insoluble carrier 2 from the opening portion of the intermediate member 30, and a silver amplification reaction was performed. The silver amplification reaction is completed in several tens of seconds.

After the completion of the silver amplification reaction, the kit was observed to determine whether the sample is positive or negative. For the determination, a capture amount was determined by imaging the extent of development of black of the anti-NP antibody coating line that increases or decreases in proportion to the NP amount by using LAS 4000 (manufactured by GE Healthcare Technologies Inc.), and calculating the signal.

Evaluation was performed based on the following standard.
E: A signal concentration is 0.045 or higher, and signals can be detected at a sufficiently high concentration.
F: The signal concentration is 0.025 to 0.045, the increase in concentration is detectable, and the presence or absence of the sample is confirmed.
G: The signal concentration is 0.015 to 0.025, which is somehow detected.
H: The signal concentration is 0.010 to 0.015, which is barely detected.
I: The signal concentration is 0.010 or less, or the concentration is not detected.

The results are shown in Table 2.

**[Table 2]**

| | Colloidal gold antibody | Membrane antibody | Concentration of SARS-CoV- 2 recombinant NP in evaluation solution (pg/ml) | | | |
|---|---|---|---|---|---|---|
| | | | 25 | 12.5 | 6.3 | 0 |
| Example 4 | Fragmented IgG2b (No1) | IgG1 (No2) | E | F | G | I |
| Example 5 | IgG2b (No1) | IgG1 (No2) | E | F | F | H |

It has been found that compared to Example 5 as an unfragmented antibody which is a colloidal gold antibody and a membrane antibody, Example 4 which is a fragmented colloidal gold antibody produces a lower signal concentration of a sample (NP concentration of 0 pg/mL) that does not contain SARS-CoV-2 in the evaluation solution and is less likely to produce false positive. In addition, it has been found that Example 4 is likely to exhibit sensitivity higher than the detection sensitivity of the SARS-CoV-2 antigen detection reagent "ESPLINE SARS-CoV-2" manufactured by FUJIREBIO Inc. even though Example 4 is fragmented.

### (Example 6)

By using the anti-NP monoclonal antibodies that belong to the subclasses IgG1 and IgG2b prepared in Example 1, an assay device was prepared according to Example 1 described in JP5430995B, and concentration was measured in the same manner as in Example 1. As a result, the effects of the present invention were confirmed as in Example 1.

### Explanation of References

1: Test strip
2: Insoluble carrier
3: Label holding pad
4: Liquid feeding pad
6: Absorbent pad
7: Back Pressure-sensitive adhesive sheet
9: Housing case
10: Upper case
12: First projection-like deformable portion
14: Second projection-like deformable portion
16: opening pore for dropwise addition of sample
18: Observation window
20: Lower case
21: Insoluble carrier-housing portion
22: Absorbent pad-housing portion
24: Second pot-housing portion
30: Intermediate member
32: First pot-housing portion
34: Breaking part
35: Flow channel forming portion
36: Back surface of flow channel forming portion 35
40: First pot
41: First amplification solution
42: Pot container
43: Sheet member
45: Second pot
46: Second amplification solution
47: Pot container
48: Sheet member
100: Immunochromatography kit
L1: Test region
L2: Confirmation region
L3: Amplification index region
[Sequence list]
International application 20F01329W1JP21041983_2.app based on the Patent Cooperation Treaty

## Claims

1. A SARS-CoV-2 detection kit for specifically detecting a nucleocapsid protein contained in a biological specimen, containing at least one antibody that specifically reacts with a SARS-CoV-2 nucleocapsid protein, the SARS-CoV-2 detection kit comprising;
a first container that houses a silver-containing compound; and
a second container that houses a reducing agent capable of reducing silver ions,
wherein the antibody includes at least one antibody that belongs to a subclass IgG2b.

2. The SARS-CoV-2 detection kit according to claim 1,
wherein the antibody is a fragmented antibody.

3. The SARS-CoV-2 detection kit according to claim 2,
wherein the fragmented antibody is an antibody prepared by a protease treatment.

4. The SARS-CoV-2 detection kit according to any one of claims 1 to 3,
wherein the antibody is labeled with a labeling substance, and
information from the labeling substance is detected by being amplified with the silver-containing compound and the reducing agent capable of reducing silver ions.

5. The SARS-CoV-2 detection kit according to claim 4,
wherein the labeling substance is colloidal gold.

6. The SARS-CoV-2 detection kit according to any one of claims 1 to 5,
wherein the reducing agent is a metal salt of Fe²⁺.

7. The SARS-CoV-2 detection kit according to any one of claims 1 to 6, further comprising:
an insoluble carrier.

8. The SARS-CoV-2 detection kit according to claim 7,
wherein the insoluble carrier has a capture region for capturing a test substance and a color developing region for detecting the reducing agent.

9. A SARS-CoV-2 detection method for specifically detecting a nucleocapsid protein contained in a biological specimen by using at least one antibody that specifically reacts with a SARS-CoV-2 nucleocapsid protein, the SARS-CoV-2 detection method comprising:
amplifying detection information of the nucleocapsid protein by using a silver-containing compound and a reducing agent capable of reducing silver ions,
wherein at least one antibody that belongs to a subclass IgG2b is used as the antibody.

10. The SARS-CoV-2 detection method according to claim 9,
wherein the antibody is a fragmented antibody.

11. The SARS-CoV-2 detection method according to claim 10,
wherein the fragmented antibody is an antibody prepared by a protease treatment.

12. The SARS-CoV-2 detection method according to any one of claims 9 to 11,
wherein the antibody is labeled with a labeling substance, and
information from the labeling substance is detected by being amplified with the silver-containing compound and the reducing agent capable of reducing silver ions.

13. The SARS-CoV-2 detection method according to claim 12,
wherein the labeling substance is colloidal gold.

14. The SARS-CoV-2 detection method according to any one of claims 9 to 13,
wherein the reducing agent is a metal salt of Fe²⁺.

15. The SARS-CoV-2 detection method according to any one of claims 9 to 14,
wherein the detection method is immunochromatography.
